# EUROPÄISCHE PATENTSCHRIFT

(11) **EP 1 837 006 B1**
(45) Veröffentlichungstag und Bekanntmachung des Hinweises auf die Patenterteilung: **06.05.2015**
(21) Anmeldenummer: 07004622.2
(22) Anmeldetag: 07.03.2007
(51) Int. Cl.: A61K 6/00

(54) **Hydroxylapatit bildendes Dentalmaterial mit bioaktiver Wirkung**
Dental material forming hydroxylapatite with bioactive effect
Hydroxyapatite formant le matériau dentaire dotée d'un effet bioactif

(30) Priorität: 23.03.2006 DE 102006013854
(43) Veröffentlichungstag der Anmeldung: 26.09.2007
(73) Patentinhaber: Heraeus Kulzer GmbH, 63450 Hanau (DE)
(72) Erfinder: Erdrich, Albert, Dr., 61231 Bad Nauheim (DE); Hoffmann, Marcus, Dr., 61250 Usingen (DE); Piotrowski, Andreas, Dr., 65582 Diez (DE)
(74) Vertreter: Bendele, Tanja

(56) Entgegenhaltungen:
- WO-A-2004/030655
- WO-A-2004/103419
- WO-A2-2005/074453
- US-A- 3 679 360
- US-A1- 2005 271 741
- TAMAKI MIYAZAKI, KANNAN SIVAPRAKASAM, JAIDEV TANTRY, RAJ SURYANARAYANAN: "Physical characterization of dibasic calcium phosphate dihydrate and anhydrate", JOURNAL OF PHARMACEUTICAL SCIENCES, vol. 98, no. 3, 18 June 2008 (2008-06-18), pages 905-916, DOI: 10.1002/jps.21443

## Beschreibung

Die Erfindung betrifft ein selbsthärtendes Hydroxylapatit bildendes 2-Komponenten-Dentalmaterial mit bioaktiver Wirkung.

Das Hartgewebe der menschlichen Zähne besteht zum überwiegenden Teil aus der anorganischen Calciumphosphatverbindung Hydroxylapatit (Ca₁₀(PO₄)₆(OH)₂). Bei einem Ersatz stehen eine Vielzahl von Füllungsmaterialien auf der Basis unterschiedlicher Werkstoffe zur Verfügung (z.B. Amalgam, Komposite, Glasiononomerzemente). Diese verhalten sich idealerweise zwar biokompatibel im Zahn, jedoch kommt es in der Regel zu keinen Interaktionen zwischen dem gesunden Zahnhartgewebe und dem Füllmaterial.

Für die kraniofaziale Chirugie werden Produkte angeboten¹, die dem menschlichen Knochenmaterial ähneln.Charakteristisch ist die Biokompatibilität, d.h. die Materialien werden durch patienteneigenen Knochen substituiert. Durch osteoklastische Resorption und Knochenneubildung findet innerhalb des Körpers eine allmähliche Umwandlung in Knochen statt.
¹Bone Source der Fa. Leibinger Stryker, Norian CRS der Fa. Synthes-Stratec

Die Pulverkomponenten bestehen bei diesen Produkten aus Dioalciumphosphat-Anhydrat (DCPA) und Tetracalciumphosphat (TTCP). Zum Anmischen der Paste wird eine Natriummonophosphatlösung verwendet. Der Zement der beiden Produkte erreicht nach dem Aushärten eine Druckfestigkeit von ungefähr 60 bzw. 30 MPa.

WO 94/20064 "Calcium Phosphate hydroxyapatite precursor and methods for making and using the same" (Erfinder L.J. Chow und S. Takagi) beschreibt einen Calciumposphatzement auf der Basis eines TTCP mit einem Ca:P-Verhältnis von <2 und eines anderen schwerlöslichen Calciumphosphatsalzes unter anderem auch DCPA. Dieser Zement wird mit einer 0.25 mmol/l H₃PO₄-Lösung angemischt und erbringt Druckfestigkeiten von 60 MPa. Ferner können Proteine. Füllstoffe, Impfkeime und Viskositätsveränderer zugesetzt sein. Das Material soll als dentales Füllmaterial, Remineratisationssubstanz, Dosensitizer und Knochenersatzmaterial geeignet sein.

WO 2004/103419 (Erfinder J. Barralet, U. Gbureck und R. Thull) betrifft ein Calciumphosphat. zement bestehend aus zwei Pulverkomponenten, wobei die erste Komponente einen Partikeldurchmesser von d₅₀(Komp. 1) < 15 µm hat und die zweite Komponente eine Partikelgröße von d₅₀(Komp2)>d₅₀(Komp1) hat. Die Komponente 2 ist 1.5 bis 10-mal so groß wie die Komponente 1. Der Anmischflüssigkeit ist eine Oligocarboxylsäure (z. B. Trinatriumcitrat. Dinatriummalat, Dinatriumtartrat) zur Herabsetzung des Zetapotentials der Partikel beigegeben, was eine bessere Anmischbarkeit von Pulver und Flüssigkeit bezweckt. In einem Beispiel wird ein Zement aus den Komponenten TTCP und DCPA genannt, dessen Pulverkomponente noch Natriumphosphat als Beschleuniger für die Abbindereaktion zugefügt wurde. Dieser Zement erhält eine hohe Druckfestigkeit (∼100 MPA). Gemäß WO 2004/103419 A wird der Flüssigkeit des Su Knochenzements eine das zeta-Potential erhöhende Substanz zugesetz, z.B. Na-Citrat, das auch als g Komplexbilder angesehen kann. Die beschriebenen Druckfestigkeiten Sind mit bis zu 100 MPa hoch.

Gemäß WO 2004 030655A1 werden die dort hergestellten Ca-Phosphatmineralien allgemein zur Wiederherstellung von "tooth material" und für Zahnzement empfohlen. Gemäss Beispiel 17 werden Monetit und Carbonat- und TCCP-haltiges Material mit NaOH/PAA zu einer Paste vermischt und gehärtet. EDTA als Komplexbildner ist in einem anderen Beispiel vorhanden. Das betreffende Beispiel 17 und folgende offenbaren jedoch nicht einen ninsiontlioh Druckfestigkeit und Abrasionsstabilität konkret geeigneten Dentalzement.

In WO 2005/074453A2 gent es um eine Zement-Zusammensetzung, bei der die Flüssigkeitskomponente eine saure, gesättigte Calcium-Phosphat Lösung ist. Die Zusammensetzung is sauer und hat anfänglich einen pH von kleiner 1.9 (vg.l Anspruch 7). Beides soll notwendig sein, um einen schnell abbindenden Zement zu erhalten.

Es stellt sich die Aufgabe ein weiteres Dentalmaterial mit einer bioaktiven Wirkung zur Verfügung zu stellen. Unter "bioaktiv" wird vornehmlich die Fähigkeit zur Remineralisierung verstanden. Ziel der Remineralisierung ist es, Hydroxylapatit [(Ca₅(PO₄)₃OH)] so abzulagern, dass die Zahnhartsubstanz ihn in ihre Struktur aufnimmt. Die Remineralisierung soll weiteren Zahnverfall verhindern und Zahnsubstanz wiederherstellen. Erfindungsgemäß wird die Aufgabe durch ein selbsthärtendes 2-Komponenten-Dentalmateiral gelöst, mit den Komponenten

### Pulverkomponente enthaltend

Dicalciumphosphat-Anhydrat (DCPA) oder Dicalciumphosphat-Dihydrat (DCPD) Tetracalciumphosphat (TTCP)
und
Flüssigkomponente enthaltend
Wasser
Komplexbildner aus der Gruppe Na₄-EDTA oder Na₅-Pentetat,
wobei kristallines DCPA mit einer für Brushit typischen Kristallgestalt als Plättchen eingesetzt wird, das einen Eisen- (Fe), Mangan- (Mn), Molybdän- (Mo) und Wolfram- (W)-Gehalt von jeweils kleiner als 0.2 m% (200 ppm) aufweist.

Die Komponenten sind zur Mischung einer Paste zur Applikation direkt in der Kavität vorgesehen. Die Paste härtet dort durch Phasenneubildung zu überwiegend Hydroxylapatit (> 95 Gew%) aus. Aufgrund der zahngleichen chemischen Zusammensetzung hat dieses Füllungsmaterial die Fähigkeit zu remineralisieren eine Fähigkeit, wie sie auch der Zahnschmelz besitzt.

Das spezielle Dicalciumphosphat-Anhydrat (DCPA) bzw. Dicalciumphosphat-Dihydrat (DCPD) der Erfindung wird für eine besonders reaktive Paste benötigt. Es wird auch bei der Synthese des eingesetzten Tetracalciumphosphats (TTCP) eingesetzt.

### Verzeichnis der Abbildungen:

- Abbildung 1, 2:: Kristallgestalt des speziellen DCPA für die Synthese des TTCPs und im Pulvergemisch der Paste.
- Abbildung 3:: Oberfläche der ausgehärteten Paste nach 24 h.
- Abbildung 4, 5:: Remineralisierte Oberfläche eines Prüfkörpers nach 44 Wochen im künstlichen Speichel (in-vitro-Versuch).
- Abbildung 6:: Füllung der ausgehärteten Paste im natürlichen Zahn nach 4 Monaten Tragezeit, Ausbildung einer remineralisierten Sicht von der Füllung zum Zahn (in-vivo-Versuch).

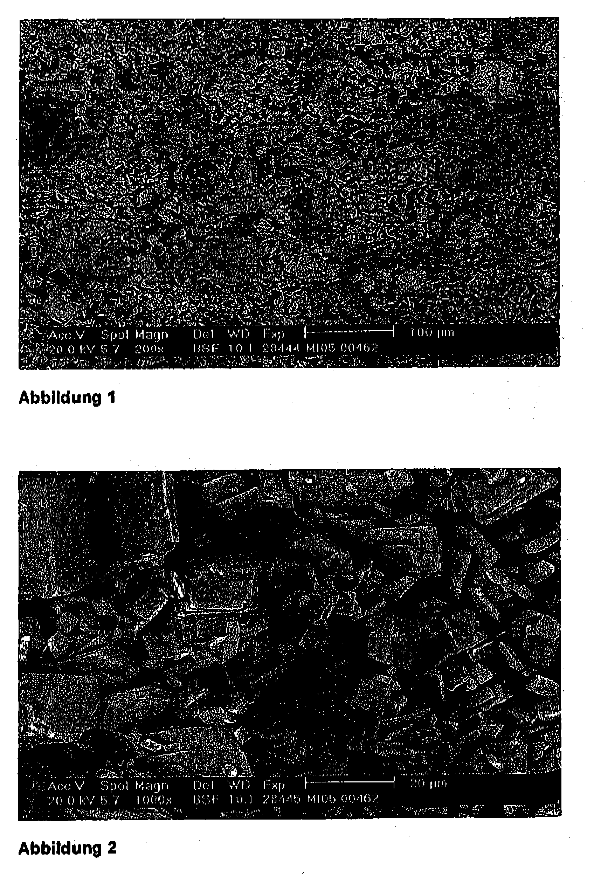

Das DCPA zeichnet sich durch eine Reinheit bezüglich der Elemente Eisen (Fe) und Mangan (Mn) aus. Der Anteil muss kleiner als 0.2m% (200ppm) betragen. Außerdem muss es sehr kristallin sein, unter Ausbildung der für Brushit typischen Kristallgestalt als Plättchen. Die Abbildungen 1 und 2 zeigen Rasterelektronenmikroskopaufnahmen der Kristallmorphologie der DCPA-Kristallite.

Die TTCP-Synthese erfolgt nach bekannten Verfahren, wie z.B. in WO9420064 beschrieben, außer, dass das oben beschriebene, besondere DCPA dafür eingesetzt wird. Ein Synthesebeispiel wird im folgenden beschrieben:

Die Paste kann durch Mischen des Pulvergemisches aus DCPA und TTCP mit einer wässrigen Lösung von Na₄-EDTA oder Na₅-Pentetat (zweckmäßig 400-700 mmol/l) gewonnen werden.

Der Zusatz der Komplexbildner erfolgt zum besseren Anmischen der Paste und zur Verarbeitung in der dentalen Anwendung (Applizieren in der Kavität, Modellieren).

Weiterhin erfolgt eine Aushärtung der Paste mit einem festeren Verbund der Hydroxylapatitpartikel als bei einem Anmischen mit einer Lösung aus Na₃-Citrat, was zwar nicht zu einer höheren Druckfestigkeit führt, aber eine höhere Abrasionsstabilität zeigt. Für die Anwendung im Bereich als direktes Füllungsmaterial ist diese Eigenschaft für eine stabile Füllung von großer Bedeutung.

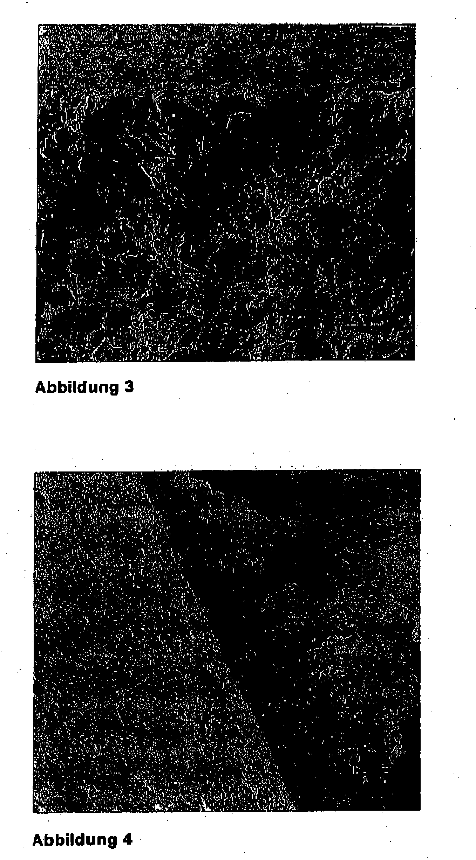

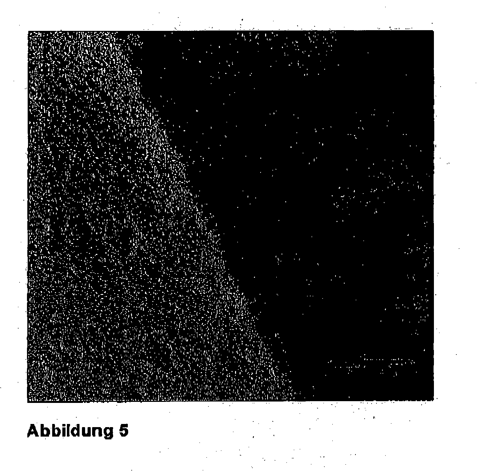

Die bioaktive Wirkung der ausgehärteten Paste konnte über in-vitro-Versuche nachgewiesen werden. Die Abbildung 3 zeigt die Oberfläche eines Prüfkörpers 24 h nach der Präparation. Nach der Lagerung der Prüfkörper in künstlichen Speichel erfolgte über eine Remineralisation eine Gefügeneubildung (Abbildungen 4 und 5). Die gestaltlosen Partikel werden zu prismatischen Strukturen umgebaut, die ähnlich dem Zahnschmelz senkrecht zur Oberfläche in den Prüfkörper "wachsen".

Ein Vorteil des erfindungsgemäßen Materials im Vergleich zu den bislang bekannten Systemen ist die hohe Druckfestigkeit in Verbindung mit einer hohen Abrasionsstabilität. Bekannte Systeme werden in erster Linie im Bereich der Knochenersatzstoffe eingesetzt, wo die Eigenschaft "Abrasionsstabilität" kein entscheidenes Kriterium ist. Jedoch erfordert die Anwendung des Materials als dentales Füllungsmaterial eine Stabilität gegenüber der Kaubelastung.

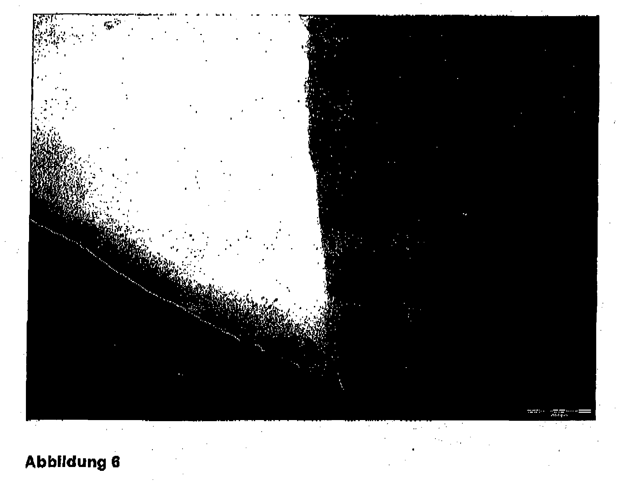

Ein weiterer Vorteil des vorliegend vorgestellten Materials ist dessen Remineralisierbarkeit. Es konnte durch in-vitro- und auch in-vivo-Experimente nachgewiesen werden, dass das Material durch die Remineralisation ein neues Gefüge bildet. Durch das formstabile Aushärten der Paste (kein Schrumpf bzw. keine Expansion) bildet sich kein Randspalt zwischen Zahn und Füllmaterial. Außerdem mineralisiert die Füllung an das gesunde Zahngewebe an (Abb. 6: Zahnschnitt 4 Monaten nach Legen der Füllung, in-vivo-Versuch).

Das folgende Beispiel zeigt eine Ausführungsform der Erfindung:

### Beispiel

Für die Synthese des TTCP wird ein DCPA mit einem Partikeldurchmesser (d₅₀) von 10-12 µm verwendet. Es wird mit Calciumcarbonat (CaCO₃) equimolar gemischt und bei 1400-1550°C für 4-18 h getempert. Nach Ablauf der Reaktionszeit wird das entstandene TTCP bei der Synthesetemperatur aus dem Ofen genommen und bei Raumtemperatur abgekühlt. Für die Verwendung im Pulvergemisch wird die Partikelgröße (d₅₀) auf einen Durchmesser von 9-18 µm durch Mahlen in einer Kugelmühle gebracht. Das DCPA im Pulvergemisch der Paste hat eine Partikelgröße von 0.5 - 3 µm und weist noch die plättchenförmige Kristallgestalt auf. Idealerweise hat das TTCP eine Partikelgröße von 10 µm und das DCPA eine Partikelgröße von 1 µm.

Durch Mischen des Pulvergemisches aus DCPA und TTCP mit einer wässrigen Lösung von Na₄-EDTA (500 mmol/l) wird eine Paste erhalten.

Nach der Aushärtung wird das Material nach ISO 9917:2004 getestet. Bei der Druckfestigkeit werden Werte von 90MPa +/- 7MPa erreicht.

Die ausgehärtete Paste zeigte eine Langzeitstabilität von über 1 Jahr.

Die Abrasionsstabilität wurde mit der ACTA-Maschine nach De Gee²³, ⁴ untersucht *(*De Gee, A.J., Pallav, P., Davidson, C.L.: Effect of abrasion medium on wear of stress-bearing composites and amalgam in vitro. J Dent Res 65, 654-658 (1986)). Hierbei zeigt die Paste der vorliegenden Erfindung zwei Drittel geringere Abrasion als das System nach WO 2004/103419.

## Patentansprüche

1. Verwendung eines selbsthärtenden Zweikomponenten-Dentalmaterials mit den Komponenten
A Pulverkomponente enthaltend
Dicalciumphosphat-Anhydrat (DCPA) oder Dicalciumphosphat-Dihydrat (DCPD),
Tetracalciumphosphat (TTCP)
und
B Flüssigkomponente enthaltend
Wasser,
Na₄-EDTA oder Na₅-Pentetat als Komplexbildner,
wobei kristallines DCPA mit einer für Brushit typischen Kristallgestalt als Plättchen eingesetzt wird, das einen Eisen- (Fe), Mangan- (Mn) Gehalt von jeweils kleiner als 200 ppm aufweist, eingesetzt wird,
als Dentalmaterial.

## Claims

1. Use of a self-curing two component dental material comprising the components
A) powder component comprising
dicalcium phosphate anhydrate (DCPA) or dicalcium phosphate dihydrate (DCPD), tetracalcium phosphate (TTCP) and
B) liquid component comprising
water,
Na₄-EDTA or Na₅-pentetate as complexing agent,
wherein crystalline DCPA with a typical crystal shape of brushite as of small plates is used, wherein iron (Fe), manganese (Mn), molybdenum (Mo) are each present in the DCPA in an amount of less than 200 ppm,
as dental material.

## Revendications

1. Utilisation d'un produit dentaire à deux composants auto-vulcanisant comportant les éléments suivants :
A) un composant en poudre contenant
phosphate dicalcique anhydrate (DCPA) ou phosphate dicalcique dihydrate (DCPD), phosphate tétracalcique (TTCP) et
B) un composant liquide contenant
eau,
Na₄-EDTA ou Na₅ pentétate comme agent complexant,
où le DCPA cristallin est utilisé avec une forme cristalline type de brushite en plaquettes, où du fer (Fe), du manganèse (Mn) et du molybdène (Mo) sont présents dans le DCPA en quantité inférieure à 200 ppm,
comme produit dentaire.
